(19)

Europäisches
Patentamt
European
Patent Office
Office européen
des brevets

(11)  **EP 2 586 365 A1**

(12)  **EUROPEAN PATENT APPLICATION**

(43) Date of publication:
     **01.05.2013 Bulletin 2013/18**

(51) Int Cl.:
     ***A61B 5/02*** *(2006.01)*        ***A61B 5/053*** *(2006.01)*

(21) Application number: **11382327.2**

(22) Date of filing: **24.10.2011**

(84) Designated Contracting States:
     **AL AT BE BG CH CY CZ DE DK EE ES FI FR GB
     GR HR HU IE IS IT LI LT LU LV MC MK MT NL NO
     PL PT RO RS SE SI SK SM TR**
     Designated Extension States:
     **BA ME**

(71) Applicant: **Universidad Politécnica de Madrid
     28040 Madrid (ES)**

(72) Inventors:
     • **Sánchez Ávila, Carmen
       E-28040 Madrid (ES)**

     • **De Santos Sierra, Alberto
       E-28040 Madrid (ES)**
     • **Bailador del Pozo, Gonzalo
       E-28040 Madrid (ES)**
     • **Guerra Casanova, Javier
       E-28040 Madrid (ES)**
     • **Jara Vera, Vicente
       E-28040 Madrid (ES)**

(74) Representative: **ABG Patentes, S.L.
     Avenida de Burgos, 16D
     Edificio Euromor
     28036 Madrid (ES)**

(54)   **Method for quantifying stress in a user**

(57)    The present invention relates to a method for quantifying stress in a user, wherein said method allows establishing discrimination between stressed users and relaxed users. The invention is characterized by the use of stress patterns based on sigmoid transfer functions to allow quantifying stress in a larger number of situations.

FIG. 1

**Description**

Object of the Invention

The present invention relates to a method for quantifying stress in a user, wherein said method allows establishing discrimination between stressed users and relaxed users. The invention is characterized by the use of stress patterns based on sigmoid transfer functions to allow quantifying stress in a larger number of situations.

Background of the Invention

Studies which seek to objectively assess the stress level in a user are known in the state of the art. For example, there is enormous interest in developing safety devices which control user access to restricted areas, for example by means of iris or fingerprint recognition or password inputting such that only authorized users have the possibility of having access. Nevertheless, the same user can seek access because he/she is violently forced or is threatened to do so. In this situation, the aforementioned safety devices should have resources which allow quantifying the stress level in preventing such coercive access attempt situations. To achieve this objective, it is necessary to have a method, in particular one that can be implemented in the safety device, which obtains a value that is a function of the stress level in the user.

The detection of stress has been considered from different viewpoints and methods of approach. For example, there are studies providing a system which assess the stress level in a user by the manner and the rate at which he/she types characters in a computer keyboard.

Other studies make use of facial recognition techniques to assess the stress level.

There are studies closer to the present invention in which the stress level assessment techniques only make use of not necessarily invasive physiological signals. This is the case of measuring finger temperature, heart rate or blood pressure.

In these cases, the physiological signals are modeled as random signals, mathematically manipulated as random variables, and therefore with an unpredictable behavior even though they are treatable by means of statistical techniques. As the present invention will do, stress level detection is sought by making use of these random signals assuming that their behavior on a statistical level changes in relation to the individual's mood. In the present invention, the random signals used are heart rate (HR) and galvanic skin response (GSR). The variables will be identified as "$h$" for heart rate and "$g$" for galvanic skin response.

These signals are easily acquired from the human body of the user by means of sensors placed on his/her fingertips and offer a direct relationship with any alteration experienced in the user, such as a mood change, being in a dangerous situation, etc. Furthermore, this easy signal acquisition allows integrating these sensors in any access device.

The response of a user to an external change modifying his/her stress level is not the same for each person. In order for the method to work properly, what will be referred to as a stress pattern or behavior pattern is used, which is simply a set of data associated with the user that allow determining the response or behavior of that user in at least two situations: relaxed and under stress. This stress pattern comprises at least one limited space of pairs, in the particular case of the present invention they will be ($h,g$), the heart rate and galvanic skin conductivity, respectively, characterizing the individual in the relaxed and stressed states. The limited space of pairs allows characterizing the individual; nevertheless, the invention makes use of the mean and a measure of dispersion as values sufficiently representative of the set of measurements for defining the pattern characterizing the individual. By means of the method according to the invention the current stress level of the individual is determined using the mentioned characterization (or pattern) and a measurement of the parameters ($h,g$). This stress level is a real value defined in a specific interval. The range of variation is typically [0,1], the null value corresponding to a situation of complete relaxation and the value 1 to a maximum stress level. Any other interval can be transformed, for example by means of linear transformation, to interval [0,1] without entailing a qualitative difference in the stress assessment.

The set of all the pairs making up the space of all the possible combinations of $h$ and $g$ is denoted as $HxG$, and each pair of points ($h$, $g$) corresponds to a sample of the heart rate $h$ and galvanic skin response $g$ signals in a specific instant.

Given a new pair ($h,g$) taken from a reading, the definition of the stress pattern establishes a function which associates a real value to said pair.

In the state of the art this function has been implemented as a sum of Gaussian membership functions within the fuzzy logic environment, i.e., each state (relaxed or stressed) has been modeled by including the behavior in one region of space $HxG$ for each state.

Despite the fact that Gaussian functions are positive functions for any value of their arguments, they rapidly decay for points of the domain located far from the maximum so for all practical purposes, for a specific threshold value, the function is considered null and therefore can be considered a support function limited and small.

Additionally, Gaussian functions are described in the state of the art by using a mean and a dispersion. It can therefore be said that the support of the function only extends to interval $[\mu - \sigma * n, \mu + \sigma * n]$ wherein $\mu$ and $\sigma$ denote the Gaussian function mean and dispersion, respectively, and $n$ denotes the number of samples where it is typically high and determined by the pre-established confidence level.

The following rules are proposed in the literature for detecting stress:

- if $(h,g) \in H_{relaxation}^{Gaussian} x G_{relaxation}^{Gaussian}$ , then stress does not exist

- if $(h,g) \in H_{stress}^{Gaussian} x G_{stress}^{Gaussian}$ , then stress exists;

wherein $SIGNAL_{function}^{state}$ corresponds with the signal used (h or g), the state can be relaxed or stress, and the functions described in the state of the art are Gaussian.

The technical problem that arises in the state of the art can mathematically be expressed as follows:

$$\left[ H_{relaxation}^{Gaussian} x G_{relaxation}^{Gaussian} \right] \cup \left[ H_{stress}^{Gaussian} x G_{stress}^{Gaussian} \right] \subset HxG$$

or in other words, the set of all the pairs included in the stress pattern is strictly contained in space $HxG$. Therefore, there will be pairs (h,g) of space $HxG$ that are not included in the stress pattern, and the stress assessment level will therefore not be possible under the conditions determined by any sample (h, g).

The technical problem of how to provide greater coverage in space $HxG$, the set of all the possible pairs of information provided by the heart rate and galvanic skin response physiological signals, so that the stress pattern more completely describes the entire possible spectrum of pairs of physiological signals arises.

The present invention according to claim 1 solves said technical problem as well as other particular problems by means of the embodiments established in the dependent claims.

Description of the Invention

The present invention mainly comprises a method for quantifying stress in a user making use of a stress pattern which allows a better description of the behavior of an individual given that it allows covering the entire spectrum of space $HxG$.

It has been seen that Gaussian membership functions are used given that it is considered that said membership functions must have a support that does not go beyond what is described by the acquired data about the behavior of the individual. This is why, for example, the support is limited to interval $[\mu - \sigma * n, \mu + \sigma * n]$. Any value outside of both states, by making use of Gaussian functions and proceeding according to the state of the art, not only is a suitable output value of the stress level not obtained, but furthermore either the value is indefinite or does not make sense given that its correspondence has not been included in the stress pattern.

To solve the technical problem raised, the invention makes use of sigmoid functions as membership functions with the particularities that will be described in detail in the detailed description of the invention, and it considers a method for quantifying stress according to claim 1. It has been experimentally found that the use of sigmoid functions allows describing the behavior of the user not only in a specific region of space $HxG$, but in its entire completeness.

The experiments corroborate that the use of such functions covers not only all of space $HxG$ but that the quantification of stress following the method according to the invention is appropriate and coherent when it is carried out in regions of space $HxG$ not described by the pairs that were initially used in constructing the stress patterns against the established belief in this field of the art. From the empirical viewpoint, it has been seen that there are regions within space $HxG$ that correspond to possible states (relaxed/stressed) and that are not covered by the stress pattern defined according to the state of the art. The steps comprised in a first aspect of the invention as well as a device adapted for carrying out said method will be described in the detailed description of the invention section.

Description of the Drawings

These and other features and advantages of the invention will be more clearly understood from the following detailed description of a preferred embodiment given only by way of nonlimiting illustrative example with reference to the attached drawings.

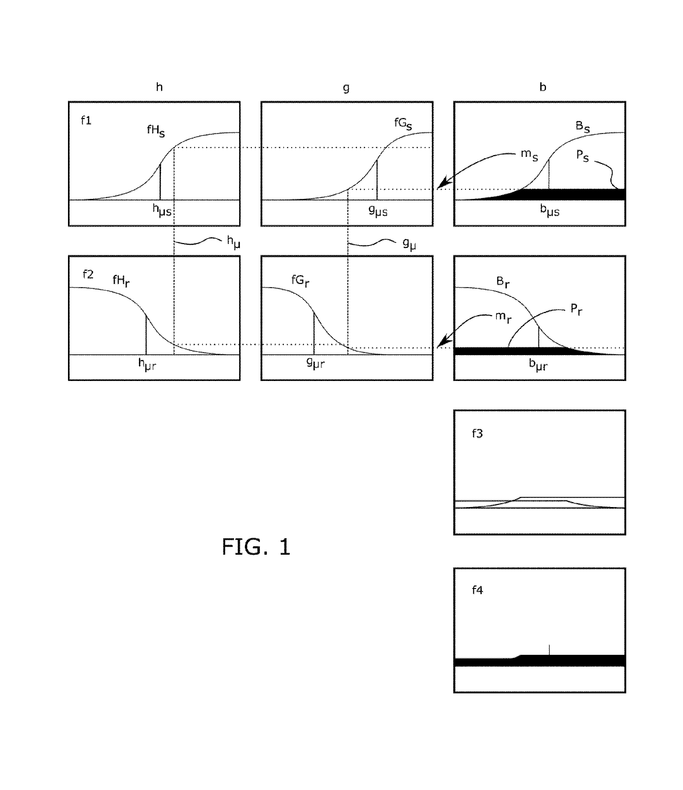

Figure 1 shows a set of functions belonging to the rules making up an embodiment of the method according to a first aspect of the invention.

The figure shows four rows of graphs distributed in three columns. The first and second rows are directly related with two stress rules, the first row is the row showing the rule corresponding to the stressed state and the second row shows the rule corresponding to the relaxed state.

The third and fourth rows only have graphs in the third column and contain the end results and the assessment mode from the previous rules.

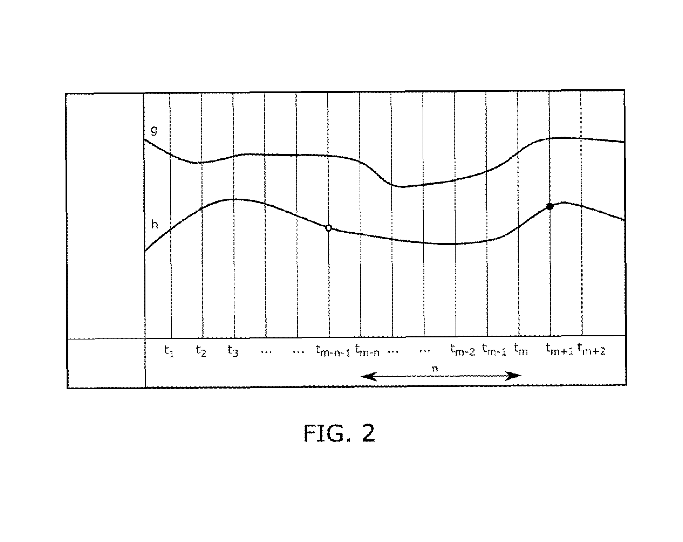

Figure 2 shows a particular way of carrying out the first aspect of the invention by performing heart rate and galvanic skin response time sampling (or galvanic skin conductivity).

Embodiments of the Invention

A first aspect of the invention is a method for quantifying stress in a user making use of a stress pattern associated with said user. The stress pattern comprises:

o a mean heart rate value ($h_{\mu r}$) and a heart rate variance value ($h_{\sigma r}$) corresponding to a relaxed state of the user,
o a mean heart rate value ($h_{\mu s}$) and a heart rate variance value ($h_{\sigma s}$) corresponding to a stressed state of the user,
o a mean galvanic skin response value ($g_{\mu r}$) and a galvanic skin response variance value ($g_{\sigma r}$) corresponding to a relaxed state of the user,
o a mean galvanic skin response value ($g_{\mu s}$) and a galvanic skin response variance value ($g_{\sigma s}$) corresponding to a stressed state of the user.

Each mean value and each variance value allow defining a sigmoid function as follows:

$$f(x, x_\mu, x_\sigma) = \frac{1}{1 + exp\left(-\frac{x - x_\mu}{x_\sigma}\right)}$$

wherein variable *x* can be either heart rate (*h*) or galvanic skin response (*g*). Value $x_\mu$ represents the mean value and value $x_\sigma$ represents the variance. This sigmoid function is increasing and the positive values above a specific threshold value exist at least to the right of the value of mean $x_\mu$ and a specific interval to the left thereof the width of which depends on the variance.

The method also takes into account decreasing sigmoid functions which adopt the following form:

$$f(x, x_\mu, x_\sigma) = 1 - \frac{1}{1 + exp\left(-\frac{x - x_\mu}{x_\sigma}\right)}$$

and therefore have positive values above a specific threshold value at least to the left of the value of mean $x_\mu$ and a specific interval to the right thereof the width of which depends on the variance.

The sum of both types of functions always have support on the entire real straight line, hence they solve the technical problem considered.

In one embodiment these values $h_{\mu r}$, $h_{\sigma r}$, $h_{\mu s}$, $h_{\sigma s}$, $g_{\mu r}$, $g_{\sigma r}$, $g_{\mu s}$, $g_{\sigma s}$ are obtained from experiments conducted on the user evaluating for each state the mean and the dispersion both of the heart rate and of the galvanic skin response. In these experiments the user is subjected to stress and relaxation conditions to measure these values in both states.

• The particular configuration of the sigmoid function is determined with these values and at least two rules are constructed, one rule associated with the relaxed state and another rule associated with stress. A rule for the relaxed state of the user is thus defined which comprises:

o constructing a decreasing function in a pre-established interval [*a*, *b*] of real values which can be expressed as:

$$fH_r\left(h, h_{\mu r}, h_{\sigma r}\right) = 1 - \frac{1}{1 + exp\left(-\frac{h - h_{\mu r}}{h_{\sigma r}}\right)}$$

o constructing a decreasing function in interval [a, b] which can be expressed as:

$$fG_r\left(g, g_{\mu r}, g_{\sigma r}\right) = 1 - \frac{1}{1 + exp\left(-\frac{g - g_{\mu r}}{g_{\sigma r}}\right)}$$

o constructing a decreasing basis function in interval [a, b] which can be expressed as:

$$B_r\left(b, b_{\mu r}, b_{\sigma r}\right) = 1 - \frac{1}{1 + exp\left(-\frac{b - b_{\mu r}}{b_{\sigma r}}\right)}$$

for predetermined values $b_{\mu r}$ and $b_{\sigma r}$.

Figure 1 shows in the second row (f2) the three functions making up the rule associated with the relaxed state from the predetermined scalars, function $fH_r$ associated with the heart rate, function $fG_r$ associated with the galvanic skin response and a basis function $B_r$ which will allow constructing a final function which establishes the value of the stress level of the user by means of a centralization measurement.

The same process is used in constructing the functions belonging to the rule associated with stress, where the latter comprises:

o constructing an increasing function in interval [a, b] which can be expressed as:

$$fH_s\left(h, h_{\mu s}, h_{\sigma s}\right) = \frac{1}{1 + exp\left(-\frac{h - h_{\mu s}}{h_{\sigma s}}\right)}$$

o constructing an increasing function in interval [a, b] which can be expressed as:

$$fG_s\left(g, g_{\mu s}, g_{\sigma s}\right) = \frac{1}{1 + exp\left(-\frac{g - g_{\mu s}}{g_{\sigma s}}\right)}$$

o constructing an increasing basis function in interval [a, b] which can be expressed as:

$$B_s\left(b, b_{\mu s}, b_{\sigma s}\right) = \frac{1}{1 + exp\left(-\frac{b - b_{\mu s}}{b_{\sigma s}}\right)}$$

In all the functions shown in Figure 1 the value zero is represented by means of a horizontal line and the point where the mean value of the sigmoid function is located is indicated. The functions of the rule associated with relaxation (f2) are decreasing and the rules associated with stress (f1) are increasing functions.

Having defined the rules associated with the relaxed and stressed states as well as the values of the reading on the user of values (h, g), the following is performed:

- the value of function $fH_r$ and $fG_r$ is determined for the heart rate ($h_\mu$) and galvanic skin response ($g_\mu$) values measured on the user corresponding to the relaxation conditions and the minimum $m_r = min\{fH_r, fG_r\}$ is determined
- the value of function $fH_s$ and $fG_s$ is determined for the heart rate ($h_\mu$) and galvanic skin response ($g_\mu$) values measured

on the user corresponding to the stress conditions and the minimum $m_s = min\{fH_s, fG_s\}$ is determined

By resorting to every technical rule, values $h_\mu$ and $g_\mu$ of pair $(h, g)$ are common, hence use has been made of a vertical line of dots to find the intersection with both functions $fH_r$ and $fG_r$ as well as functions $fH_s$ and $fG_s$.

By resorting to function $fH_r$ with the value of $h_\mu$ the value adopted by said function is seen with a horizontal line of more spaced out dots. By resorting to function $fG_r$ with the value of $g_\mu$, it is found that the value obtained is less. It can be seen that the horizontal line going towards the right, also represented with more spaced out dots, is lower. The calculation of the minimum selects this second value as that which will be used in the basis function.

The same occurs in the rule associated with the stress, the value measured on function $fH_s$ is greater than the value measured on function $fG_s$ and therefore it is also the second value (the value which verifies the minimum condition) that is used in the basis function.

Basis functions are used for constructing what is referred to as weight functions, doing so in a similar manner for either technical rule:

- the weight function $P_{r(x)} = min\{m_r, Br(x, b_{\mu r}, b_{\sigma r})\}$ is defined for any value of $x$ in interval $[a, b]$,
- the weight function $P_{s(x)} = min\{m_s, B_s(x, b_{\mu s}, b_{\sigma s})\}$ is defined for any value of $x$ in interval $[a, b]$,

Once the minimum in the variable heart rate and galvanic skin response has been determined, the basis function is truncated, maintaining only the values that are below that minimum value. In the third column, where the basis functions are represented, it is observed that under the value represented by the line of spaced out dots the area of the basis function is shown with a black background. This area in black corresponds to the construction of the weight function both for the relaxed state $P_{r(x)}$ and the stressed state $P_{s(x)}$. The minimum condition between the previously calculated minimum value and the basis function has that effect of truncating the basis function.

The next step consists of constructing a result function $P(x)$ as $P(x) = max\{Pr(x), Ps(s)\}$ for any value of $x$ in interval $[a, b]$.

In the third column, where the basis functions are represented, there are two areas with a black background, one for the rule corresponding to the relaxed state and the other for the rule corresponding to a stress situation. The two profiles are superimposed in the third row (f3). The result of the maximum function of the step which constructs the result function is a new function, the profile of which follows the highest points of the superposition of the weight functions. The same result function is shown in the fourth row (f4) where it is now shown with the area under the curve in black and constructed by means of the maximum condition.

Finally, the stress quantification value in the user is determined by evaluating a centralization measurement of function $P(x)$ in interval $[a, b]$.

In the experiments conducted, interval $[0,1]$ has been used as a particular case for interval $[a, b]$ and parameters $b_{\mu r}, b_{\sigma r}, b_{\mu s}, b_{\sigma s}$ take the value 1.

Even though in this simplest example only two rules corresponding to the relaxed state and to the stressed state have been used, it is possible to combine the use of three or more rules. In this second case, two situations are distinguished:

○ if the rule corresponds to the relaxed state, said rule has a decreasing sigmoid function for the heart rate, a decreasing sigmoid function for the galvanic skin response and a decreasing sigmoid basis function,
○ if the rule corresponds to the stressed state, said rule has an increasing sigmoid function for the heart rate, an increasing sigmoid function for the galvanic skin response and an increasing sigmoid basis function.

The manner of proceeding in these cases is the same as in the case described in the first embodiment. The minimum of the sigmoid function corresponding to the heart rate value ($h_\mu$) and of the sigmoid function corresponding to the galvanic skin response value ($g_\mu$), respectively, both measured on the user, is determined for every defined rule. The weight function is also determined for every technical rule as the minimum function between the previous minimum value and the basis function of the technical rule; and, finally the result function $P(x)$ is assessed as the maximum both of the weight functions of the rule for the relaxed state and the rule for the stressed state as well as the weight functions of the additional rules.

In a final step, the centralization measurement of the weight function obtained in the previous step in interval $[a, b]$ is assessed as a stress quantification value in the user.

In the experiments conducted it has been observed that there are groups of users with similar features. In other words, given a certain population, it is possible to define groups having close parameters in the rules defining their stress pattern. These groups are formed by individuals whose heart rate response and galvanic skin response have a similar or close behavior given the same stressing stimuli. A stress pattern can thus be pre-assigned to users from different groups of people.

As an experimental test, a collection of stress patterns which serve as typical patterns has been elaborated such that it is possible to pre-assign a stress pattern to a person without having to initially carry out a heart rate and galvanic skin response assessment task by subjecting the user to stress and relaxation situations. This prior assignment is not known in the state of the art and prevents an expensive and time-consuming operation.

In the preferred embodiment of the invention the pre-assignment of a typical stress pattern is done by means of a form adapted for determining user's behavior with respect to stress. This assessment, contrasted with the responses obtained making use of the same test in the individuals used for elaborating the typical patterns, allows defining the correspondence between an individual and his/her pre-assigned group with the closest stress pattern.

An embodiment of a test and the generation of typical patterns from this test will be described at the end of this description of examples section.

Another object of this invention is the continuous measurement of heart rate and galvanic skin response values over time. The measurements of the heart rate ($h_\mu$) and of the galvanic skin response ($g_\mu$) of the user is carried out by calculating the arithmetic mean on a set of samples taken over a time period.

When an individual already has a pre-assigned stress pattern, the application of the method of the invention allows quantifying the stress. Given that this pre-assignment is very generic and each individual is pre-assigned a stress pattern from a group of "typical" users, it is possible to improve the stress pattern over time in order to gradually define it individually and characterize it with specific and more accurate heart rate and galvanic skin response values for that user. In addition, once the pattern is established, preferably if it has been improved with measurements on the user, the measurements of the heart rate and galvanic skin response allow analyzing the evolution of the stress level of the user over time. The improvement of the stress patterns makes sense both when the first assigned pattern corresponds to a pre-classification by "typical users" and because the behavior of an individual with respect to external stressing stimuli also evolves over time.

Figure 2 shows a graph resulting from the reading over time in an individual of the physiological signals to be used for correcting the stress pattern. This graph shows the two variables, heart rate ($h$) and galvanic skin response ($g$). Graphs are obtained for the two states, stressed and relaxed, (even though only one of them is shown by way of example) so four curves will be obtained: $h^s$, $h^r$, $g^s$, $g^r$. This time evolution is acquired by measuring the heart rate and the galvanic skin response of the user over time by means of continuous control methods, for example, measuring them using a keyboard or mouse monitoring the heart rate and the galvanic skin response. This is how the method acquires values, those which are experimentally measured, which allow characterizing the pattern more precisely because it takes into account both the real response of the user and the evolution of his/her response over time.

As described in claims 5 and 6, in this embodiment the time variable has been discretized in equal time intervals, and making use of this discretization, a sampling of $m$ samples is carried out over time in instants $t_i$, wherein $i = 1...m$, both of the heart rate ($h$) and of the measurement of the galvanic skin response ($g$) of the user.

A subset of n consecutive samples comprising the sample taken in instant $t_m$ and the previous consecutive samples wherein $n < m$ is taken. In other words, the n consecutive samples in instant $t_m$ and in n-1 previous instants ($t_{m-n}$, ..., $t_{m-1}$) are selected. Figure 2 shows the extension in the time variable reached by the sampling of these $n$ samples by means of an arrow.

The arithmetic mean is evaluated on the subset of $n$ samples to obtain the measurements both of the heart rate ($h_\mu$) and of the galvanic skin response ($g_\mu$) of the user. The value is evaluated on the subset of n samples to obtain the measures of dispersion both of the heart rate ($h_\sigma$) and of the galvanic skin response ($g_\sigma$) of the user. Values $h_\mu$, $h_\sigma$, $g_\mu$, $g_\sigma$ thus correspond to a measurement averaged over time.

The evolution of these averaged variables requires maintaining the sampling both of the heart rate ($h$) and of the measurement of the galvanic skin response ($g$) of the user.

Figure 2 shows the next sample taken in instant $t_{m+1}$ in variable $h$ by means of a black circular area. A new collection of $n$ consecutive samples is constructed starting from that of $n$ previous samples resulting from adding the sample taken in instant $t_{m+1}$ and eliminating the oldest sample taken in instant $t_{m-n}$ which is shown in Figure 2 by means of a hollow circle.

Therefore, by following a more generic manner of proceeding, the evolution of the quantification of stress is assessed by taking as consecutive measurements both of the heart rate ($h_\mu$) and of the galvanic skin response ($g_\mu$) of the user those measurements which result from calculating the arithmetic mean on the subset of samples which results from gradually adding to the subset of $n$ samples a specific number of samples obtained in the following instants of time and in turn discarding the same number of older measurements.

This final method for assessing heart rate and galvanic skin response over time has been described as one that allows determining the mean on $n$ last samples. These same arguments serve to measure the measure of dispersion. These measurements must be conducted in both the relaxed and stressed states. It is not possible to simultaneously take a measurement on the same individual in the relaxed and stressed states. This means that the preceding method can be interpreted in two different ways.

A first interpretation carries out the correction over time of the measurements of the mean and the standard deviation for the heart rate and the galvanic response corresponding to one of the states without modifying the parameters characterizing the other state; and in a later time period the correction over time of the parameters corresponding to that other state is carried out. A second interpretation establishes that the discretization over time makes use of very broad time intervals, for example each $t_i$ corresponds to a different day, such that in that time period it is possible to subject the individual to two different states and that the measurements are estimated as if they correspond to the same instant of time.

As described above, a first way to determine the stress pattern consists of carrying out on the individual direct measurements both of the heart rate and of the galvanic skin response. The previous method allows weighting several measurements in a time period which lead to values that are the closest possible to the real behavior of the individual with respect to stress. It has also been described that to at first avoid subjecting the individual to such tests, it is possible to pre-assign a pattern by subjecting the individual to a test that does not require measuring heart rate and galvanic response. An embodiment of such test for pre-assigning said pattern to a user is described below.

This test defines three possible aspects: Cognitive, physiological and motor. A set of questions for the user is used for the test; 22 situations which involve the three aspects to be measured (cognitive, physiological and motor) will be used in this embodiment:

1. Before an exam in which I have a lot at stake or if I am gong to be interviewed for an important job
2. When I am going to be late for an appointment or date
3. When I think about all the things I have to do
4. When making a decision or solving a difficult problem
5. At work or when I study
6. When I am waiting for someone in a crowded place
7. If someone of the opposite sex is very close to me, lightly touching me, or if I am in an intimate sexual situation
8. When someone bothers me or when I argue
9. When I am watched or my job is supervised, when I receive criticism, or whenever I may be negatively evaluated
10. If I have to speak in public
11. When I think about recent experiences where I have felt ridiculous, shy, humiliated, alone or rejected
12. When I have to take a plane or a boat
13. After having made a mistake
14. Before a dentist appointment, injections, wounds or blood.
15. When I am on a date with someone of the opposite sex
16. When I think about my future or about future difficulties or problems
17. In the middle of crowds or in enclosed spaces
18. When I have to attend a social gathering or meet new people
19. In high places or deep waters
20. When observing violent scenes
21. Noting specifically
22. When it is time to go to sleep

The following responses are presented for these possible situations, depending on the factor to be evaluated.

Cognitive Aspect

1. I easily worry
2. I have negative thoughts or feelings about myself, such as "inferior" to others, clumsy, etc...
3. I feel unsure of myself
4. I think about things too much without actually making a decision
5. I am afraid
6. It's hard to concentrate
7. I think that people will notice my problems or my awkward acts

Physiological Aspect

1. I have an upset stomach

2. My hands or another part of my body sweats even on cold days
3. My hands or legs shake
4. My head hurts
5. My body is tense
6. I have palpitations, my heart beats very fast
7. I feel like I'm suffocating and my breathing is agitated
8. I feel nauseous or dizzy
9. My mouth becomes dry and it's hard for me to swallow
10. I have chills and shiver even though it is not that could

Motor Aspect

1. I cry easily
2. I make repetitive movements with some part of my body (scratch myself, touch myself, rhythmic movements with my feet or hands, etc...)
3. I smoke, drink or eat too much
4. I try to avoid or run away from the situation
5. I move or do things without a specific purpose
6. I become paralyzed or my movements are awkward
7. I stutter or have other verbal expression difficulties

The test requires selecting one and only one response of each aspect to be measured (cognitive, physiological and motor) for each of the 22 situations.

The scores corresponding to the physiological, motor and cognitive aspects are constructed from these responses

Use is made of a total value which is calculated as C+F/2+M is, and also of four partial values grouping the scores of the 22 situations in four groups. These four groups are referred to as factors I to IV (in Roman numerals). The following formulas are used to obtain the factors from I to IV, where S1, ..., S22, are situations 1 to 22.

- F-I: S1+S4+S8+S10+S11+S13

- F-II: S7+S15+S18

- F-III: S12+S14+S17+S19

- F-IV: S5+S21+S22

The following is a detailed description of what each of the aspects used in the score of each situation includes:

- cognitive: characterized by responses such as fear of being negatively evaluated, difficulties in concentrating, uncertainty or difficulty in making decisions;
- physiological: characterized by high saturations of responses relating to physiological aspects such as dizziness, nausea, chills, etc...
- motor activation: characterized by activities which relate to performing repetitive movements, scratching oneself, smoking or drinking in excess, etc..

And concerning the four factors also obtained from the test scores:

- Factor I: Situations involving evaluation and taking on responsibilities

- Factor II: Sexual and social interaction situations

- Factor III: Phobic situations

- Factor IV: Common or everyday life situations.

This test in two ways, first to establish the groups sharing a common characterization pattern; and a second used to assign a previously determined pattern to a user.

To obtain a set of groups of users who share a common pattern, a number of users who have been subjected to the test while at the same time taking measurements both of the heart rate and of the galvanic skin response to determine real individual patterns is selected.

It is thus possible to create a table where each row of that table corresponds to each of the users who has been subjected both to the test and to the physiological tests where each score obtained in the previous factors is therefore related with the physiological signal dispersions and mean values, making a one-to-one association between user, scores and physiological values.

Then a clustering algorithm, specifically a k-means clustering algorithm, is produced, grouping all the individuals in 10 possible clusters. The more users that have served as a basis for performing the tests the higher the number of clusters and therefore of "typical" patterns that will result from these experiments will be.

Each cluster will contain a representative value of the factors relating to stress and a representative value of each physiological value. Therefore, given certain stress scores, those physiological values the responses of which to the stress test more accurately correspond with the stress scores provided will be associated with it.

80 people have been used in this embodiment to yield 10 clusters and therefore 10 typical patterns:

| | Cognitiv | Physio. | Motor | Total | F1 | F2 | F3 | F4 |
|---|---|---|---|---|---|---|---|---|
| 1 | 73 | 63.5 | 76 | 212.5 | 85 | 24 | 36 | 25 |
| 2 | 67 | 23 | 31 | 121 | 54 | 19 | 4 | 33 |
| 3 | 83 | 58 | 96 | 237 | 137 | 40 | 21 | 26 |
| 4 | 134 | 89 | 102 | 325 | 154 | 39 | 87 | 25 |
| 5 | 124 | 72.5 | 58 | 254.5 | 96 | 34 | 89 | 54 |
| 6 | 79 | 17.5 | 39 | 135.5 | 74 | 18 | 8 | 13 |
| 7 | 95 | 75 | 78 | 248 | 140 | 28 | 69 | 23 |
| 8 | 57 | 48.5 | 65 | 170.5 | 78 | 22 | 59 | 5 |
| 9 | 59 | 17.5 | 65 | 141.5 | 54 | 18 | 20 | 13 |
| 10 | 105 | 57.5 | 38 | 200.5 | 91 | 26 | 63 | 18 |
| | Scores | | | | | | | |

In the first row of the table, in the header, "Cognitiv" identifies the score given for the aspect cognitive, "Physio." identifies the score given for the physiological aspect and "Motor" identifies the score given for the aspect relating to motor activation which has resulted in each of the patterns after carrying out the clustering. Rows 1 to 10 show the data for each of the 10 patterns obtained in this embodiment. The "Total" column is the total sum of the score; and columns F1, F2, F3 and F4 are the grouped sums of the scores which led to each of the factors I, II, III and IV.

For the same patterns, the mean and dispersion values for the heart rate and galvanic skin response for the relaxed state are also represented:

| | mean HR | HR dev | mean SC | SC dev |
|---|---|---|---|---|
| 1 | 76 | 10 | 6 | 1 |
| 2 | 87 | 12 | 10 | 2 |
| 3 | 100 | 15 | 5 | 1 |
| 4 | 98 | 11 | 7 | 3 |
| 5 | 93 | 17 | 9 | 2 |
| 6 | 89 | 16 | 14 | 2 |
| 7 | 81 | 10 | 8 | 1 |
| 8 | 94 | 12 | 9 | 3 |
| 9 | 102 | 14 | 10 | 2 |
| 10 | 95 | 14 | 12 | 2 |
| | Relaxed | | | |

and for the stressed state:

|  | mean HR | HR dev | mean SC | SC dev |
|---|---|---|---|---|
| 1 | 120 | 8 | 14 | 7 |
| 2 | 110 | 9 | 17 | 6 |
| 3 | 112 | 13 | 8 | 5 |
| 4 | 108 | 11 | 9 | 5 |
| 5 | 110 | 14 | 10 | 3 |
| 6 | 105 | 11 | 16 | 5 |
| 7 | 109 | 15 | 11 | 6 |
| 8 | 114 | 13 | 8 | 4 |
| 9 | 123 | 10 | 5 | 5 |
| 10 | 112 | 9 | 3 | 4 |
|  | Stress | | | |

In this first step, the test has served to define the "typical" patterns. Once these typical patterns are defined, the same test serves to obtain a particular score on a user; and, by comparing the Euclidean distance between factors F1, F2, F3 and F4 with those obtained in the definition of the typical patterns, the closest pattern is established as the pattern pre-assigned to said user.

Another object of this invention is a device adapted for quantifying stress in a user according to claim 8.

## Claims

1. A method for quantifying stress in a user which comprises the following steps:

    • obtaining a stress pattern for the user which in turn comprises:

        o a mean heart rate value ($h_{\mu r}$) and a heart rate variance value ($h_{\sigma r}$) corresponding to a relaxed state of the user,
        o a mean heart rate value ($h_{\mu s}$) and a heart rate variance value ($h_{\sigma s}$) corresponding to a stressed state of the user,
        o a mean galvanic skin response value ($g_{\mu r}$) and a galvanic skin response variance value ($g_{\sigma r}$) corresponding to a relaxed state of the user,
        o a mean galvanic skin response value ($g_{\mu s}$) and a galvanic skin response variance value ($g_{\sigma s}$) corresponding to a stressed state of the user,

    • measuring the heart rate ($h_{\mu}$) and the galvanic skin response ($g_{\mu}$) of the user in whom stress is to be quantified,
    • obtaining at least one rule for the relaxed state of the user which comprises:

        o defining a decreasing function in an interval [$a$, $b$] of real values which can be expressed as:

$$fH_r\left(h, h_{\mu r}, h_{\sigma r}\right) = 1 - \frac{1}{1 + exp\left(-\frac{h - h_{\mu r}}{h_{\sigma r}}\right)}$$

        o defining a decreasing function in the interval [$a$, $b$] which can be expressed as:

$$fG_r\left(g, g_{\mu r}, g_{\sigma r}\right) = 1 - \frac{1}{1 + exp\left(-\frac{g - g_{\mu r}}{g_{\sigma r}}\right)}$$

        o defining a decreasing basis function in the interval [$a$, $b$] which can be expressed as:

$$B_r(b, b_{\mu r}, b_{\sigma r}) = 1 - \frac{1}{1 + exp\left(-\frac{b - b_{\mu r}}{b_{\sigma r}}\right)}$$

for predetermined values $b_{\mu}r$ and $b_{\sigma r}$,

• obtaining at least one rule for the stressed state of the user which comprises:

  o defining an increasing function in the interval [*a*, *b*] which can be expressed as:

$$fH_s(h, h_{\mu s}, h_{\sigma s}) = \frac{1}{1 + exp\left(-\frac{h - h_{\mu s}}{h_{\sigma s}}\right)}$$

  o defining an increasing function in the interval [*a*, *b*] which can be expressed as:

$$fG_s(g, g_{\mu s}, g_{\sigma s}) = \frac{1}{1 + exp\left(-\frac{g - g_{\mu s}}{g_{\sigma s}}\right)}$$

  o defining an increasing basis function in the interval [*a*, *b*] which can be expressed as:

$$B_s(b, b_{\mu s}, b_{\sigma s}) = \frac{1}{1 + exp\left(-\frac{b - b_{\mu s}}{b_{\sigma s}}\right)}$$

for predetermined values $b_{\mu s}$ and $b_{\sigma s}$,

• determining the value of the function $fH_r$ and $fG_r$ for the heart rate ($h_\mu$) and galvanic skin response ($g_\mu$) values measured on the user and determining the minimum

$$m_r = min\{fH_r, fG_r\}$$

• determining the value of function $fH_s$ and $fG_s$ for the heart rate ($h_\mu$) and galvanic skin response ($g_\mu$) values measured on the user and determining the minimum $m_s = min\{fH_s, fG_s\}$
• defining the weight function $P_{r(x)} = min\{m_r, Br(x, b_{\mu r}, b_{\sigma r})\}$ for any value of *x* in the interval [*a*, *b*],
• defining the weight function $P_{s(x)} = min\{m_s, B_s(x, b_{\mu s}, b_{\sigma s})\}$ for any value of *x* in the interval [*a*, *b*],
• defining the result function $P(x) = max\{Pr(x), Ps(x)\}$ for any value of *x* in the interval [*a*, *b*],
• obtaining a centralization measurement of the function $P(x)$ in the interval [*a*, *b*] as a stress quantification value in the user.

2. The method according to claim 1, **characterized in that** the interval [*a*, *b*] is the interval [0,1].

3. The method according to claim 2, **characterized in that** $b_{\mu r}$, $b_{\sigma r}$, $b_{\mu s}$, $b_{\sigma s}$ take the value 1.

4. The method according to claim 1, **characterized in that** it additionally incorporates:

  • one or more rules corresponding to the relaxed state, to the stressed state or rules for both states, wherein:

  o if the rule corresponds to the relaxed state, said rule has a decreasing sigmoid function for the heart rate, a decreasing sigmoid function for the galvanic skin response and a decreasing sigmoid basis function,
  o if the rule corresponds to the stressed state, said rule has an increasing sigmoid function for the heart rate, an increasing sigmoid function for the galvanic skin response and an increasing sigmoid basis function,

• the minimum of the sigmoid function corresponding to the heart rate value ($h_\mu$) and of the sigmoid function corresponding to the galvanic skin response value ($g_\mu$) respectively measured on the user are determined for every defined rule,
• determining the weight function for every technical rule as the minimum function between the previous minimum value and the basis function of the technical rule; and,
• the result function $P(x)$ is assessed as the maximum of both the weight functions of the rule for the relaxed state and the rule for the stressed state and the weight functions of the additional rules.
• providing the centralization measurement of the weight function obtained in the previous step in interval [$a$, $b$] as a stress quantification value in the user

5.  The method according to claim 1, **characterized in that** the measurement of the heart rate ($h_\mu$) and the measurement of the galvanic skin response ($g_\mu$) of the user are carried out by calculating the arithmetic mean on a set of samples taken over a time period.

6.  A method for determining a stress pattern, **characterized in that**:

• for each of the states, the relaxed state and the stressed state, a sampling of $m$ samples is carried out over time in instants $t_i$, wherein $i$ = 1...$m$, both of the heart rate ($h$) and of the measurement of the galvanic skin response ($g$) of the user,
• a subset of $n$ consecutive samples is taken comprising the sample taken in the instant $t_m$ and the previous consecutive samples wherein $n < m$,
• the arithmetic mean is evaluated on the subset of $n$ samples for obtaining the measurements both of the heart rate ($h_\mu$) and of the galvanic skin response ($g_\mu$) of the user,
• the variance is evaluated on the subset of $n$ samples for obtaining the measures of dispersion both of the heart rate ($h_\sigma$) and of the galvanic skin response ($g_\sigma$) of the user,
• the evolution of the quantification of the stress is assessed by taking as consecutive measurements both of the heart rate ($h_\mu, h_\sigma$) and of the galvanic skin response ($g_\mu, g_\sigma$) of the user those which result from calculating the arithmetic mean on the subset of samples which results from gradually adding to the subset of $n$ samples a specific number of samples obtained in the following instants of time and in turn discarding the same number of older measurements.

7.  A method for determining a stress pattern for an individual, **characterized in that**:

a) a number of users in whom a test is carried out for determining the individual's response to stress by determining one or more numerical factors characterizing it is taken,
b) empirically determining the stress pattern for each individual,
c) carrying out a clustering process determining a number of "typical" patterns representative of each group resulting from the clustering,
d) for the individual, subjecting him to the test used in step a) and finding the closest numerical factors corresponding to the groups obtained in the clustering according to the Euclidean measure and pre-assigning the pattern of the closest group to the individual.

8.  A device for quantifying stress in a user comprising:

• a first sensor for detecting the heart rate signal,
• a second sensor for detecting the galvanic skin response signal,
• a central processing unit in communication with the first and second sensor adapted for carrying out a method according to any of claims 1 to 7,
• an output in communication with the central processing unit for obtaining the stress quantification value.

FIG. 1

FIG. 2

**EP 2 586 365 A1**

Europäisches
Patentamt
European
Patent Office
Office européen
des brevets

# EUROPEAN SEARCH REPORT

Application Number

EP 11 38 2327

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ES 2 361 686 A1 (UNIV MADRID POLITECNICA [ES]; SECUWARE S N; SECUWARE S L) 21 June 2011 (2011-06-21) * abstract; claims 1-10; figures 1-6 * | 1-5,8 | INV. A61B5/02 A61B5/053 |
| A | US 2002/123704 A1 (HORI KUNIHIKO [JP] ET AL) 5 September 2002 (2002-09-05) * paragraphs [0049] - [0052]; claims 1-13; figures 4-11 * | 1,8 | |
| A | US 2010/022852 A1 (WESTERINK JOANNE HENRIETTE DESIREE MONIQUE [NL] ET AL) 28 January 2010 (2010-01-28) * paragraphs [0020] - [0034]; claims 1-19; figures 1-3 * | 1,8 | |

TECHNICAL FIELDS
SEARCHED (IPC)

A61B

~~The present search report has been drawn up for all claims~~

| Place of search | Date of completion of the search | Examiner |
|---|---|---|
| Munich | 7 March 2012 | Apostol, Simona |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-5(completely); 8(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 11 38 2327

The Search Division considers that the present European patent application does not comply with the
requirements of unity of invention and relates to several inventions or groups of inventions, namely:

    1. claims: 1-5(completely); 8(partially)

        Method for quantifying stress as centralization measurement
        of a function contructed by using a specific algorithm
        comprising sigmoid based rules and weighting functions
                             ---

    2. claims: 6(completely); 8(partially)

        A method for determining a stress pattern by calculating
        statistical variability of heart rate and galvanic skin
        response over time in temporal subsets
                             ---

    3. claims: 7(completely); 8(partially)

        A method for determining a stress pattern by carring out a
        test and a clustering process
                             ---

## ANNEX TO THE EUROPEAN SEARCH REPORT
## ON EUROPEAN PATENT APPLICATION NO.

EP 11 38 2327

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2012

| Patent document cited in search report | | | Publication date | Patent family member(s) | | | Publication date |
|---|---|---|---|---|---|---|---|
| ES 2361686 | A1 | | 21-06-2011 | NONE | | | |
| US 2002123704 | A1 | | 05-09-2002 | KR | 20020070855 | A | 11-09-2002 |
| | | | | TW | 510789 | B | 21-11-2002 |
| | | | | US | 2002123704 | A1 | 05-09-2002 |
| | | | | US | 2005137503 | A1 | 23-06-2005 |
| US 2010022852 | A1 | | 28-01-2010 | CN | 101610716 | A | 23-12-2009 |
| | | | | EP | 2120701 | A1 | 25-11-2009 |
| | | | | US | 2010022852 | A1 | 28-01-2010 |
| | | | | WO | 2008099320 | A1 | 21-08-2008 |

EPO FORM P0459

For more details about this annex : see Official Journal of the European Patent Office, No. 12/82